(19) Europäisches Patentamt
European Patent Office
Office européen des brevets

(11) Publication number: **0 542 284 A2**

(12) **EUROPEAN PATENT APPLICATION**

(21) Application number: **92119392.6**

(22) Date of filing: **12.11.92**

(51) Int. Cl.[5]: **C07D 487/04**, //(C07D487/04, 249:00,231:00)

(30) Priority: **12.11.91 JP 322379/91**

(43) Date of publication of application: **19.05.93 Bulletin 93/20**

(84) Designated Contracting States: **DE FR GB NL**

(71) Applicant: **KONICA CORPORATION**
**26-2, Nishi-shinjuku 1-chome**
**Shinjuku-ku**
**Tokyo(JP)**

(72) Inventor: **Onda, Hiroyuki, Konica Corpoartion**
**1, sakura-machi**
**Hino-shi, Tokyo(JP)**
Inventor: **Ota,Katsuzi, Konica Corpoartion**
**1, sakura-machi**
**Hino-shi, Tokyo(JP)**

(74) Representative: **Henkel, Feiler, Hänzel & Partner**
**Möhlstrasse 37**
**W-8000 München 80 (DE)**

(54) **Method for producing pyrazolo [3,2-c] -1,2,4-triazole compounds.**

(57) A method for producing a pyrazolo[3,2-c]-1,2,4-triazole compound of high purity from a thiadiazine compound is provided. In the manufacture of the triazole compound represented by Formula [II] or Formula [III] from the thiadiazine compound represented by Formula [I], by-product sulfur formed in the process of manufacture is removed by recrystallizing the product from a recrystallizing solvent containing an aromatic hydrocarbon solvent including benzene, toluene, xylene, chlorobenzene and nitrobenzene.

Formula [I]

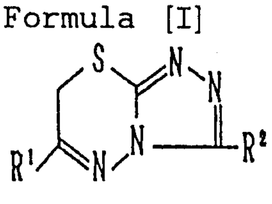

Formula [II]

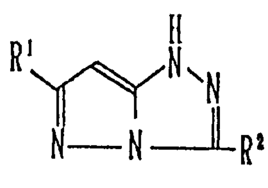

Formula [III]

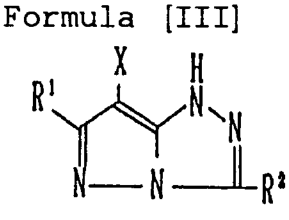

EP 0 542 284 A2

## FIELD OF THE INVENTION

The present invention relates to a method for producing pyrazolo[3,2-c]-1,2,4-triazole compounds, more specifically to a method for producing pyrazolo[3,2-c]-1,2,4-triazole compounds in high purity by use of 1,2,4-triazolo[3,4-b]-1,3,4-thiadiazine compounds as intermediates.

## BACKGROUND OF THE INVENTION

Pyrazolo[3,2-c]-1,2,4-triazole compounds are useful as photographic couplers, particularly magenta couplers, or intermediates thereof or as intermediates in organic syntheses.

These pyrazolo[3,2-c]-1,2,4-triazole compounds are synthesized by use of 1,2,4-triazolo[3,4-b]-1,3,4-thiadiazine compounds as intermediates, forming sulfur as a by-product.

This by-product sulfur is involved in the formation of other by-products in the processes that follow. As a result, troubles arise in the course of synthesis, for example, purification of the final products becomes extremely difficult and yields of the intermediates or objective compounds are lowered. Further, when light-sensitive materials are made using a compound containing sulfur as a coupler, light-sensitive emulsions are subjected to fatal influences including deterioration in sensitivity.

For eliminating these problems without lowering the yield in each process and exerting no adverse effects on the objective compounds, there have been attempted various methods for removing the sulfur in a simple manner and obtaining the intermediates or objective compounds in a high purity. So far, however, sufficient results have not been obtained.

For example, Japanese Pat. Appl. No. 145610/1990 discloses a method for producing pyrazolo[3,2-c]-1,2,4-triazole compounds which comprises removal of sulfur by use of a borohydride salt. But, this method has a disadvantage of requiring an additional post-treatment.

In view of the above situation, the present inventors made an intensive study and have accomplished the present invention by finding out that a pyrazolo[3,2-c]-1,2,4-triazole compound can be obtained in a high purity by recrystallizing an arbitrary intermediate subjected to desulfurizing reaction from a recrystallizing solvent containing at least one aromatic hydrocarbon solvent, in the process to obtain an objective compound represented by Formula [II] or Formula [III]. The above finding is an outcome of a large number of sulfur processing techniques accumulated through various tests and experiments made by the present inventors.

## SUMMARY OF THE INVENTION

The object of the present invention is to provide a new method for producing a highly pure pyrazolo-[3,2-c]-1,2,4-triazole compound useful as coupler, or intermediate thereof, represented by Formula [II] or [III] or as intermediate in organic syntheses by use of 1,2,4-triazolo[3,4-b]-1,3,4-thiadiazine compounds as intermediate.

The above object of the invention is achieved by removing the sulfur, which is formed in some of a plural number of processes to produce a pyrazolo[3,2-c]-1,2,4-triazole compound represented by Formula [II] or [III] using a 1,2,4-triazolo[3,4-b]-1,3,4-thiadiazine compound represented by Formula [I] as an intermediate, through recrystallization from a recrystallizing solvent containing at least one aromatic hydrocarbon solvent.

Formula [I]

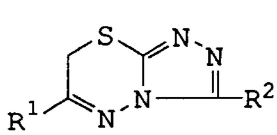

In Formula [I], $R^1$ and $R^2$ each represent a hydrogen atom or a substituent.

Formula [II]

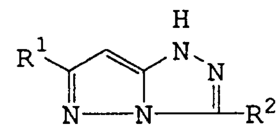

In Formula [II], $R^1$ and $R^2$ are the same as $R^1$ and $R^2$ in Formula [I].
Formula [III]

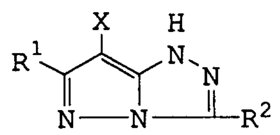

In Formula [III], $R^1$ and $R^2$ are the same as $R^1$ and $R^2$ in Formula [I], X represents a substituent capable of being split upon reaction with an oxidation product of a color developing agent.

## DETAILED DESCRIPTION OF THE INVENTION

The invention is hereinafter described in detail.

First, Formula [I] is further described.

In the 1,2,4-triazolo[3,4-b]-1,3,4-thiadiazine compound represented by Formula [I], $R^1$ and $R^2$ each represent a hydrogen atom or a substituent. The substituent represented by $R^1$ or $R^2$ is not particularly limited, but it is typically an alkyl, aryl, anilino, acylamino, sulfonamido, alkylthio, arylthio, alkenyl or cycloalkyl group. Other substituent examples include a halogen atom and a cycloalkenyl, alkynyl, heterocyclic, sulfonyl, sulfinyl, phosphonyl, acyl, carbamoyl, sulfamoyl, cyano, alkoxy, aryloxy, heterocycloxy, siloxy, acyloxy, carbamoyloxy, amino, alkylamino, imido, ureido, sulfamoylamino, alkoxycarbonylamino, aryloxycarbonylamino, alkoxycarbonyl, aryloxycarbonyl, and heterocyclothio groups. Further, a spiro-compound residue and a bridged hydrocarbon compound residue are also included.

The alkyl group represented by $R^1$ or $R^2$ is preferably one having 1 to 32 carbon atoms, which may be either straight-chained or branched.

The aryl group represented by $R^1$ or $R^2$ is preferably a phenyl group.

The acylamino group represented by $R^1$ or $R^2$ includes an alkylcarbonylamino, and arylcarbonylamino group. The sulfonamido group represented by $R^1$ or $R^2$ include an alkylsulfonylamino and arylsulfonylamino group.

The alkyl component of the alkylthio or arylthio group represented by $R^1$ or $R^2$ includes the alkyl and aryl group represented by $R^1$ or $R^2$.

The alkenyl group represented by $R^1$ or $R^2$ is preferably one having 2 to 32 carbon atoms, the cycloalkyl group so-represented is preferably one having 3 to 12, particularly 5 to 7 carbon atoms, and the alkenyl group may be straight-chained or branched.

The cycloalkenyl group represented by $R^1$ or $R^2$ is preferably one having 3 to 12, particularly 5 to 7 carbon atoms.

As other examples of the substituent represented by $R^1$ or $R^2$, the sulfonyl group includes an alkylsulfonyl and arylsulfonyl group; the sulfinyl group includes an alkylsulfinyl and arylsulfinyl group; the phosphonyl group includes an alkylphosphonyl, alkoxyphosphonyl, aryloxyphosphonyl and arylphosphonyl group; the acyl group includes an alkylcarbonyl and arylcarbonyl group; the carbamoyl group includes an alkylcarbamoyl and arylcarbamoyl group; the sulfamoyl group includes an alkylsulfamoyl and arylsulfamoyl group; the acyloxy group includes an alkylcarbonyloxy and arylcarbonyloxy group; the carbamoyloxy group includes an alkylcarbamoyloxy and arylcarbamoyloxy group; the ureido group includes an alkylureido and arylureido group; the sulfamoyl amino group includes an alkylsulfamoyl and arylsulfamoyl amino group; the heterocyclic group is preferably a 5- to 7-membered one, typically a 2-furyl, 2-thienyl, 2-pyrimidinyl or 2-benzothiazolyl group; the heterocycloxy group is preferably a 5- to 7-membered one such as a 3,4,5,6-tetrahydropyranyl-2-oxy or 1-phenyltetrazole-5-oxy group; the heterocyclothio group is preferably a 5- to 7-membered heterocyclothio group such as a 2-pyridinylthio, 2-benzothiazolylthio or 2,4-diphenoxy-1,3,5-triazole-6-thio group; the siloxy group includes a trimethylsiloxy, triethylsiloxy and dimethylbutylsiloxy group; the imido group includes a succinimido, 3-heptadecylsuccinimido, phthalimido and glutarimido group; the spiro-compound residue includes a spiro[3,3]heptane-1-yl; and

the bridged hydrocarbon compound residue includes a bicyclo[2,2,1]heptane-1-yl, tricyclo[3,3,1,$1^{3.7}$]-decane-1-yl and 7,7-dimethylbicyclo[2,2,1]heptane-1-yl.

In Formula [II] representing the pyrazolo[3,2-c]-1,2,4-triazole compound produced according to the invention, $R^1$ and $R^2$ are defined in the same way as $R^1$ and $R^2$ in Formula [I].

Further, in Formula [III] representing the pyrazolo[3,2-c]-1,2,4-triazole compound produced according to the invention, $R^1$ and $R^2$ defined in the same way as $R^1$ and $R^2$ in Formula [I].

X represents a group capable of being split upon reaction with an oxidation product of a color developing agent.

Examples of such a group are a halogen atom (e.g., a chlorine, bromine or fluorine atom) and an alkoxy, aryloxy, heterocycloxy, acyloxy, sulfonyloxy, alkoxycarbonyloxy, aryloxycarbonyl, alkyloxalyloxy, alkoxyoxalyloxy, alkylthio, arylthio, heterocyclothio, alkyloxythiocarbonylthio, acylamino, sulfonamido, N-atom-bonded nitrogen-containing heterocycle, alkyloxycarbonylamino, aryloxycarbonylamino, and carboxyl group.

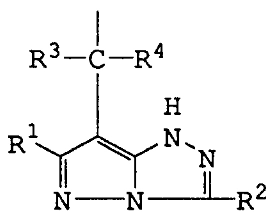

($R^1$ and $R^2$ are the same as $R^1$ and $R^2$ of Formula [I]; $R^3$ and $R^4$ each represent a hydrogen atom, an aryl, alkyl or hetero cyclic group).

Preferably, X is a halogen atom, especially a chlorine atom.

There can be used various solvents as the aromatic hydrocarbon solvent for carrying out the recrystallization which constitutes a characteristic feature of the invention. Examples of such solvents include benzene, toluene, xylene, chlorobenzene, trichlorobenzene, nitrobenzene. Of them, benzene, toluene and xylene are preferred; toluene is particularly preferred. These aromatic hydrocarbon solvents may also be a mixture of two or more different types.

The recrystallization according to the invention can be carried out by the method desribed in "Zoku Jikken Kagaku Koza (Second Series of Lectures on Chemical Experiments)", vol. 2, edited by the Chemical Society of Japan, published by Maruzen Co., 1967. To be concrete, the dissolving temperature is in a range of 20°C to 200°C, preferably in a range of 40°C to 150°C in view of the dissolving rate of the compound and the heat stability of mother nuclei. The solvent is used in an amount at which the pyrazolo[3,2-c]-1,2,4-triazole compound forms a supersaturated state; the amount of the solvent is in a range of preferably 0.1 to 100 times, especially 1 to 20 times the amount of the compound. The aromatic hydrocarbon solvent for the recrystallization according to the invention may contain other arbitrary solvents at arbitrary mixing ratios.

Examples of those solvents which can be mixed in the aromatic hydrocarbon solvent include aliphatic hydrocarbons such as n-hexane, cyclohexane, petroleum ether, ligroin; esters such as ethyl acetate; alcohols such as methanol, ethanol, isopropanol; ethers such as ethyl ether, isopropyl ether, tetrahydrofuran; halogenated hydrocarbons such as methylene chloride, chloroform; ketones such as acetone, methyl ethyl ketone; nitriles such as acetonitrile; and water.

In preparing the compound represented by Formula [II] or Formula [III] in the invention (hereinafter occasionally abbreviated as compound [II] or compound [III]) by use of the compound represented by Formula [I] (hereinafter occasionally abbreviated as compound [I]) as intermediate, there can be used a method, for example, comprising the desulfurization of compound [I] under heating. But, preferably, compound [II] and compound [III] are produced by the following path:

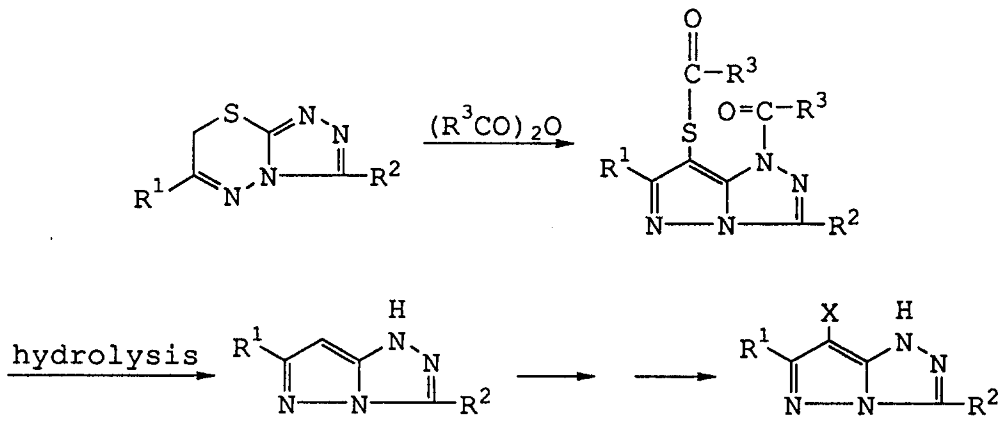


In the above formulas, $R^3$ represents an alkyl, cycloalkyl or aryl group. The alkyl group represented by $R^3$ includes the methyl, ethyl, propyl, butyl, heptyl, undecanyl, tridecanyl, trifluoromethyl, monochloromethyl and 1-ethylheptyl groups. The cycloalkyl group includes a cyclopropyl, cyclobutyl, cyclopentyl and cyclohexyl group. The aryl group includes a phenyl group.

The compound represented by Formula [I] may be allowed to react with a neat acid anhydride of Formula [IV] in the above reaction equation, or it may be allowed to react in the form of a dispersion of a solvent. Solvents usable in the reaction according to the invention are typically hydrocarbons, benzenes, ethers and halogenated hydrocarbons.

Among these, aliphatic hydrocarbons and aromatic hydrocarbons are preferred. The reaction temperature is preferably 30°C to 200°C, especially 40°C to 150°C, in view of the reaction rate and the heat stability of the mother nuclei.

In the above reaction equation, the compound represented by Formula [V] is hydrolyzed by use of an acid such as hydrochloric acid, sulfuric acid or acetic acid.

Solvent usable in the hydrolysis are typically ethers and alcohols. Particularly preferred are alcohols such as methanol, ethanol, isopropanol, etc.

The hydrolysis temperature is preferably 30°C to 200°C, especially 40°C to 150°C, in view of the hydrolysis rate and heat stability of the mother nuclei.

The compound represented by Formula [II] contaminated by sulfur as an impurity. Therefore, the sulfur is removed, in the invention, in an arbitrary process among the processes to form compound [III] from compound [II].

That is, the sulfur is removed by recrystallizing an arbitrary intermediate after the formation of compound [II], or an objective compound, from a recrystallizing solvent containing at least one of the aromatic hydrocarbon solvents.

The following are typical examples of the pyrazolo[3, 2-c]-1,2,4-triazole compound of Formula [II] produced according to the method of the invention; but, these are a limited number of exemplifications, and the compounds obtained by the invention are by no means limited to these exemplified compounds.

[II]-1

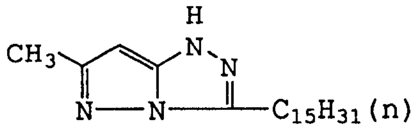

[II]-2

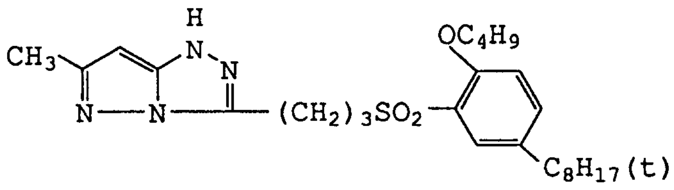

[II]-3

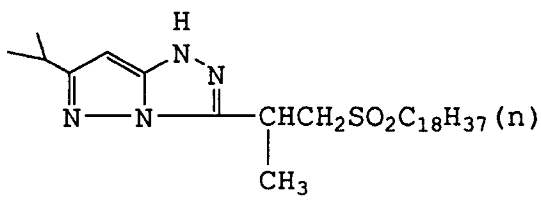

[II]-4

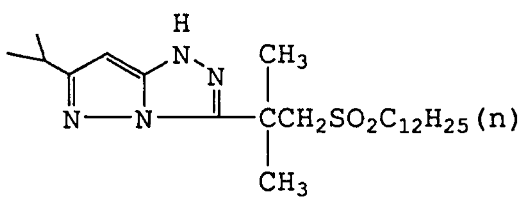

[II]-5

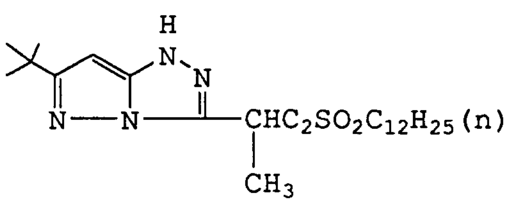

[II]-6

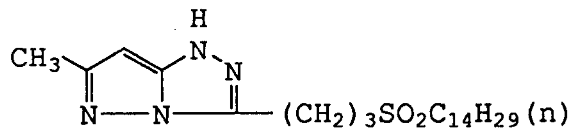

[II]-7

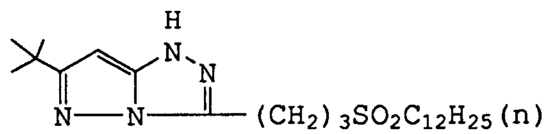

[II]-8

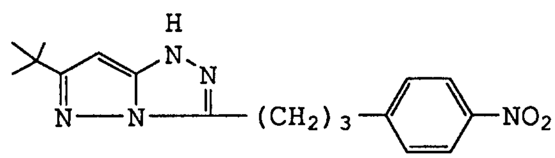

[II]-9

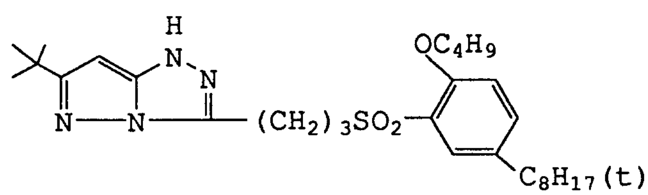

[II]-10

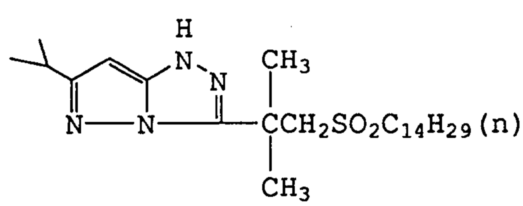

[II]-11

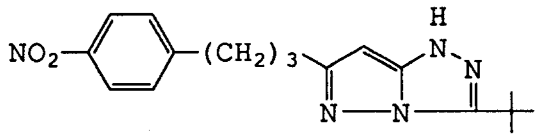

[II]-12

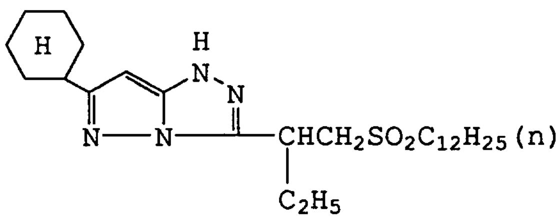

[II]-13

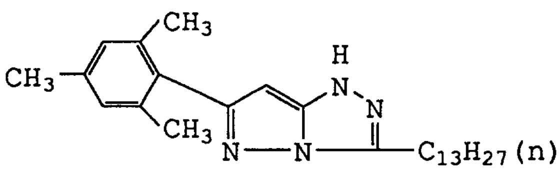

[II]-14

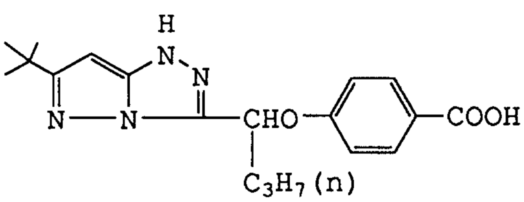

[II]-15

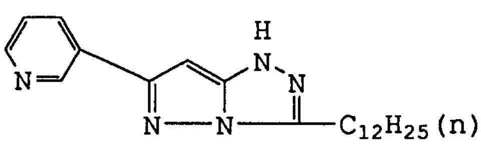

[II]-16

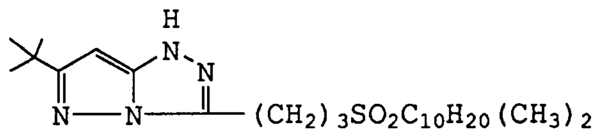

[II]-17

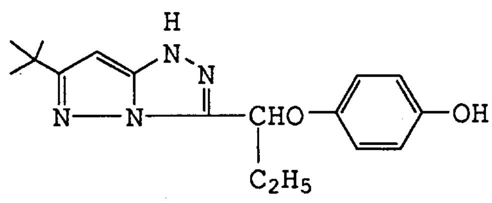

[II]-18

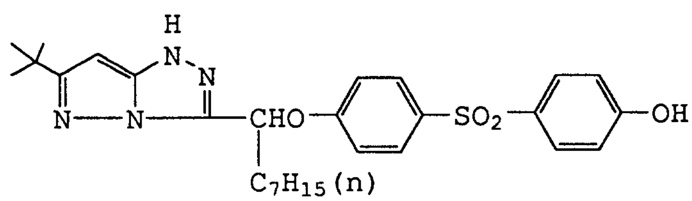

In the above structures,

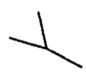

represents $(CH_3)_2CH-$ and

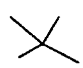

represents $(CH_3)_3C-$.

The following are typical examples of the pyrazolo[3, 2−c]−1,2,4−triazole compound of Formula [III] produced according to the method of the invention; but, these are a limited number of exemplifications, and the compounds obtained by the invention are not limited to these exemplified compounds.

[III]-1

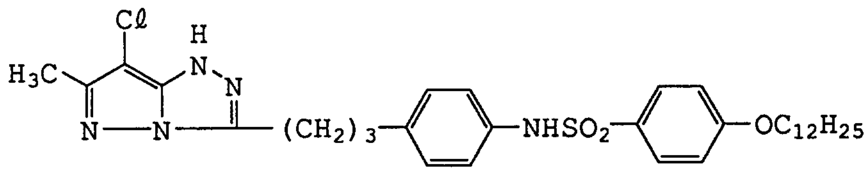

[III]-2

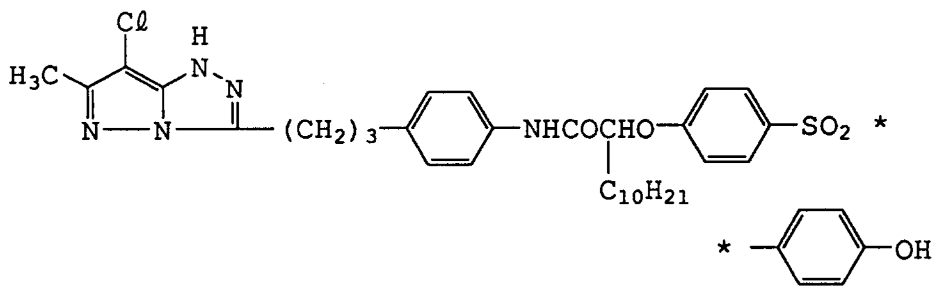

[III]-3

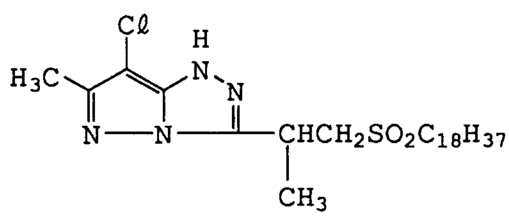

[III]-4

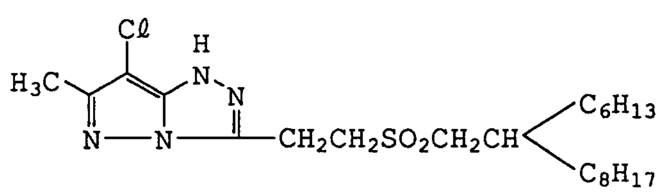

[III]-5

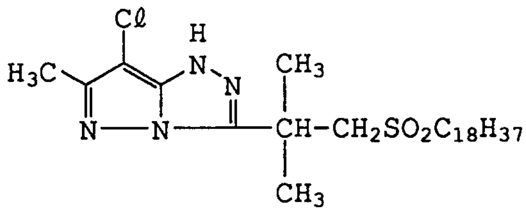

[III]-6

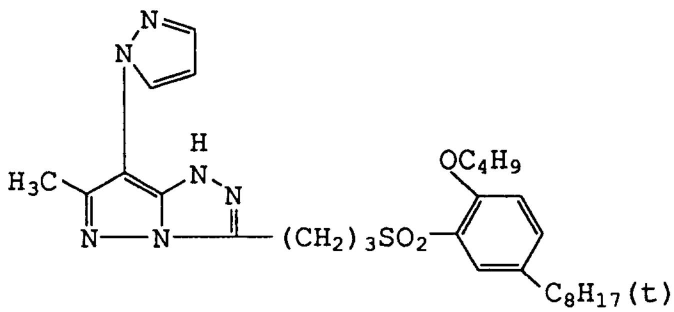

[III]-7

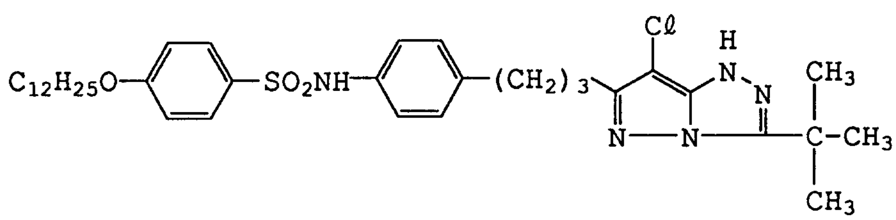

[III]-8

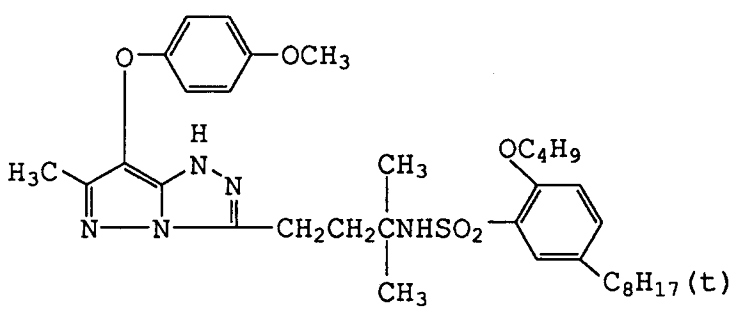

[III]-9

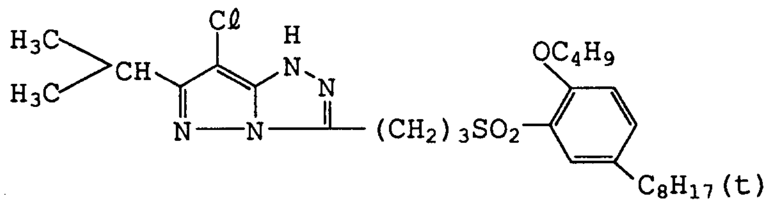

[III]-10

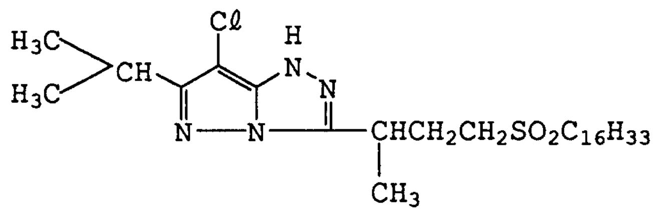

[III]-11

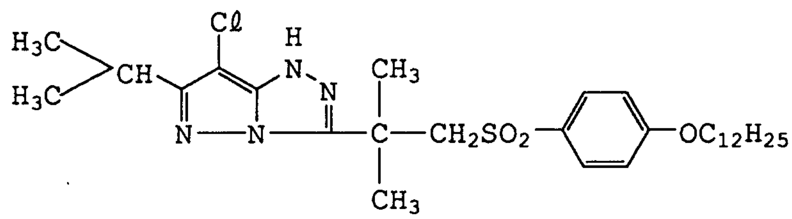

[III]-12

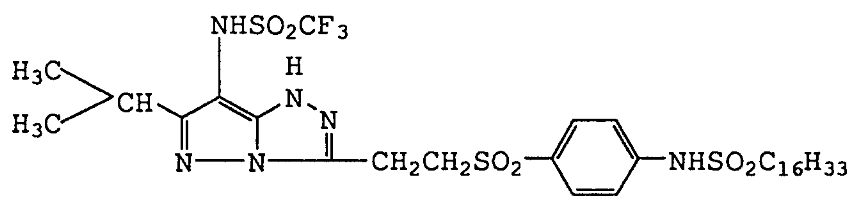

[III]-13

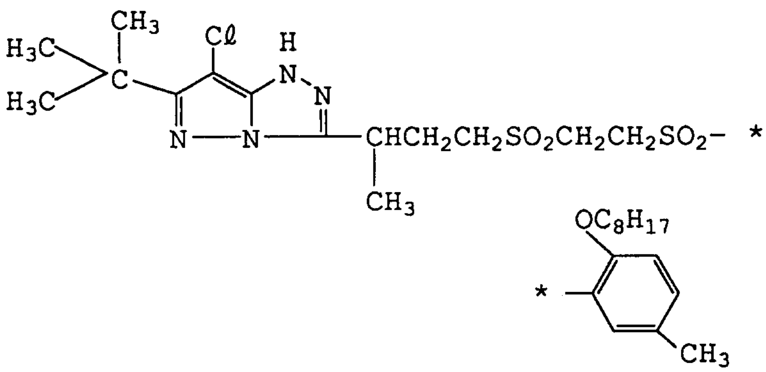

[III]-14

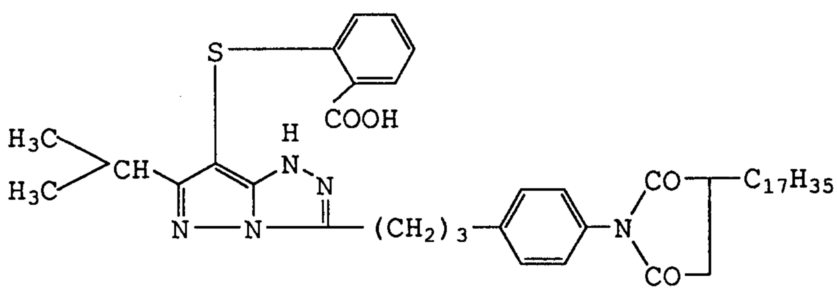

[III]-15

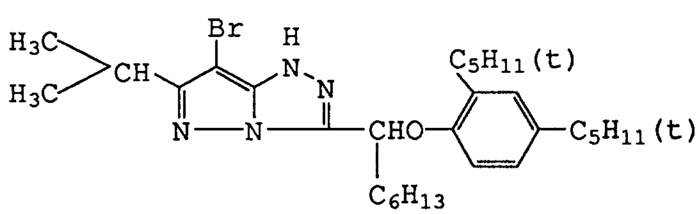

[III]-16

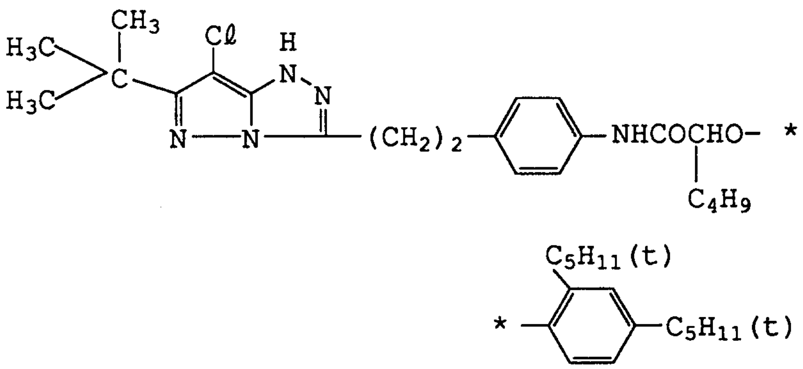

[III]-17

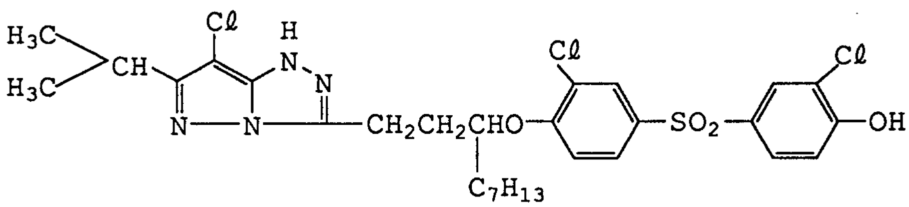

[III]-18

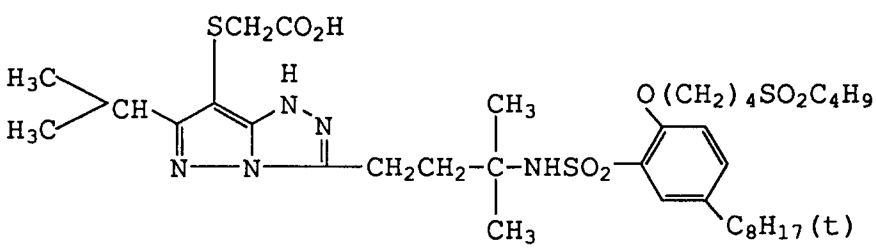

[III]-19

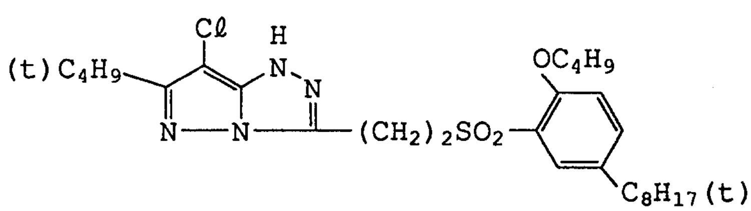

[III]-20

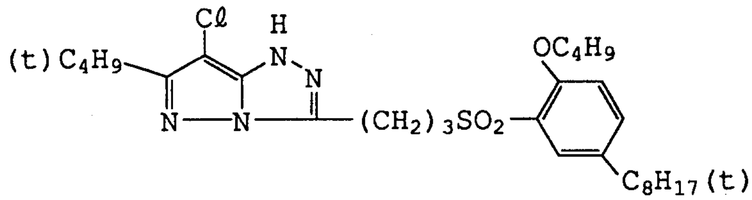

[III]-21

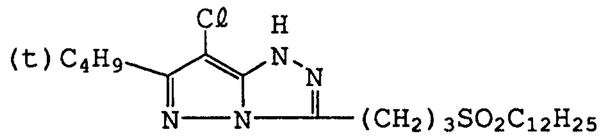

[III]-22

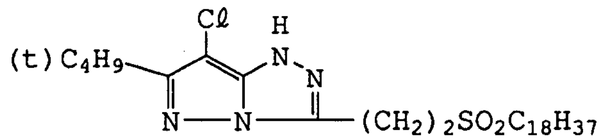

[III]-23

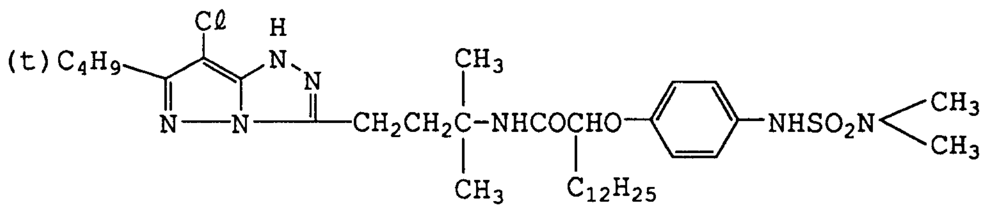

[III]-24

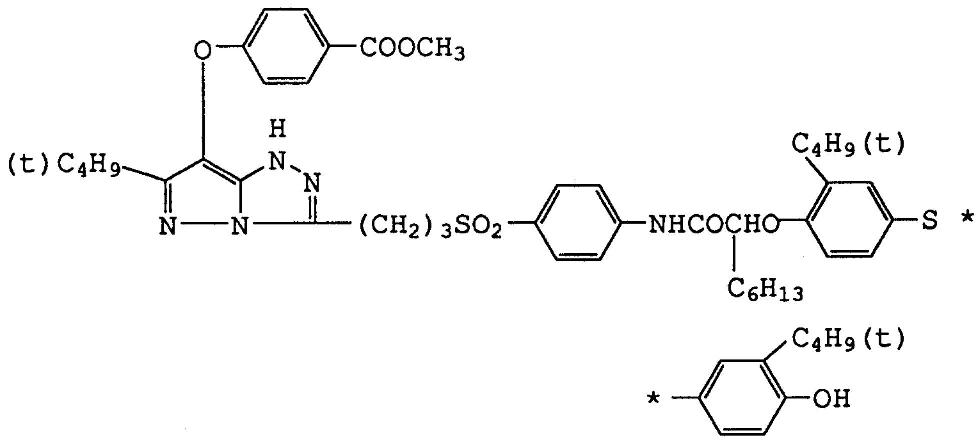

[III]-25

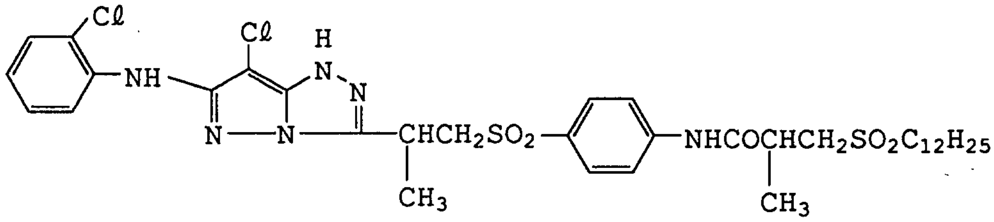

[III]-26

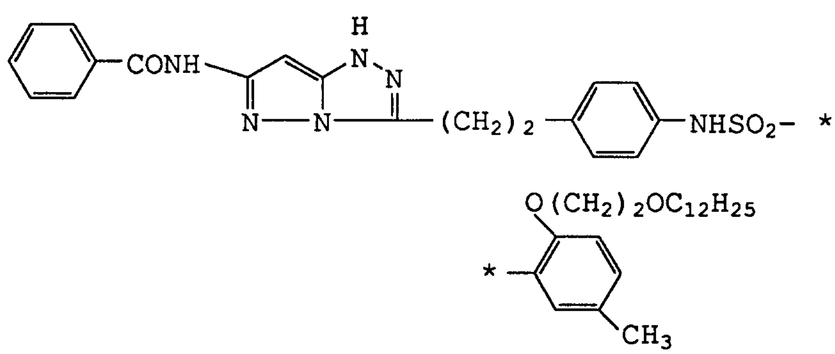

[III]-27

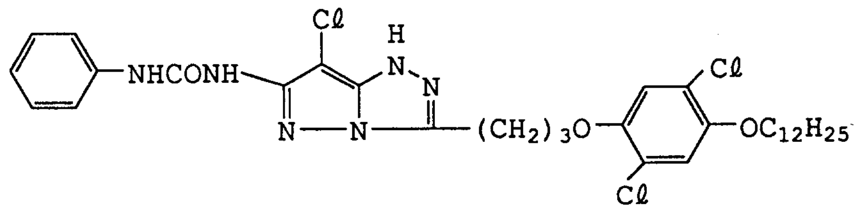

[III]-28

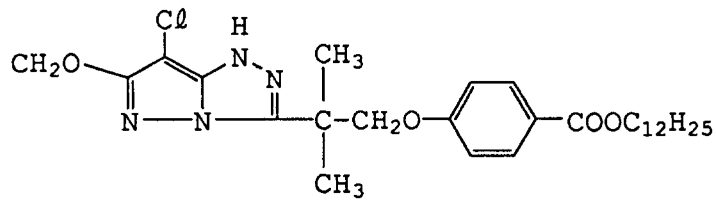

[III]-29

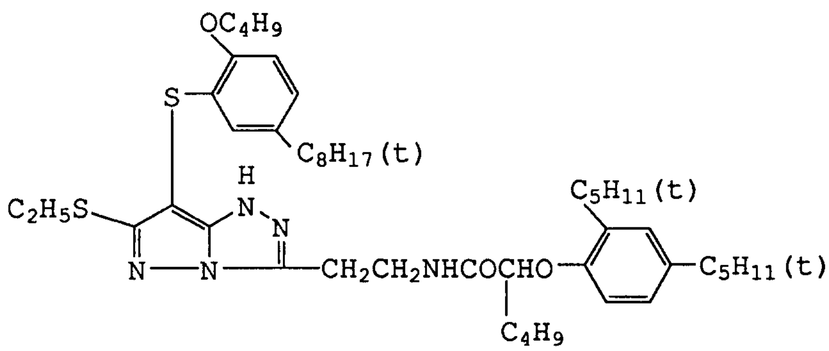

[III]-30

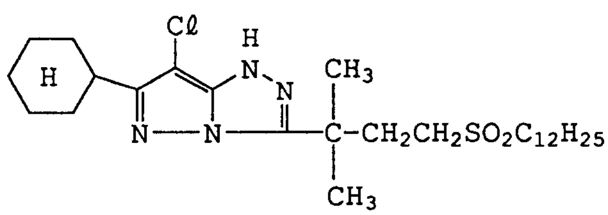

[III]-31

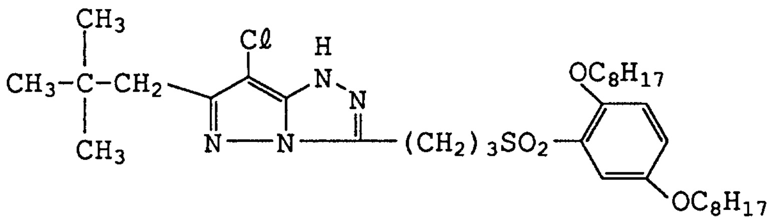

[III]-32

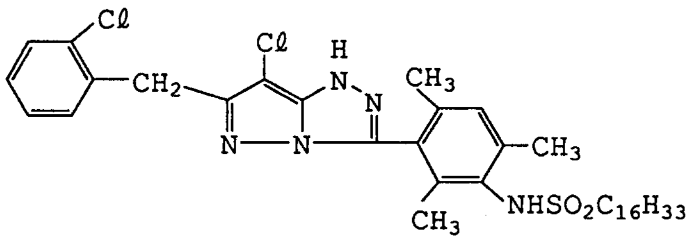

[III]-33

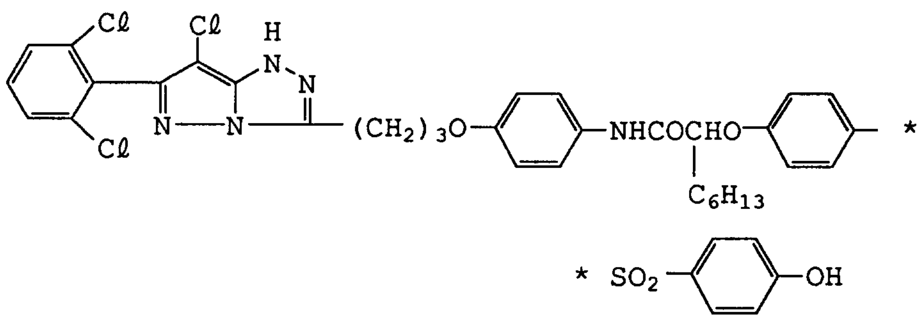

[III]-34

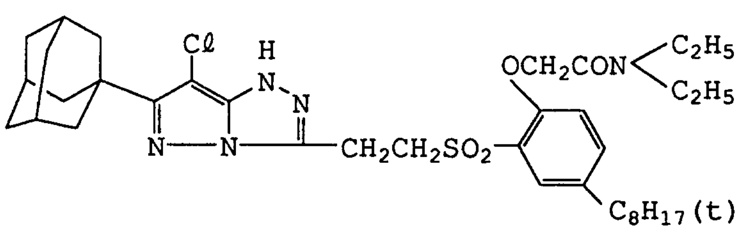

## EXAMPLES

Typical embodiments of the invention are shown hereunder. But, as a matter of course, the invention is not limited to these embodiments.

Synthesis Example 1

Compound [II] and compound [III] were synthesized by the preferred reaction steps afore-mentioned by use of compound [I] as intermediate.

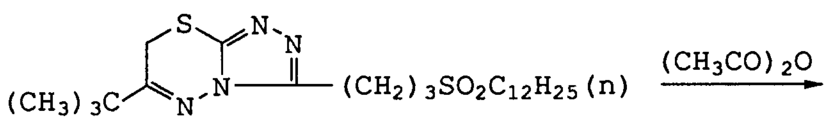


A

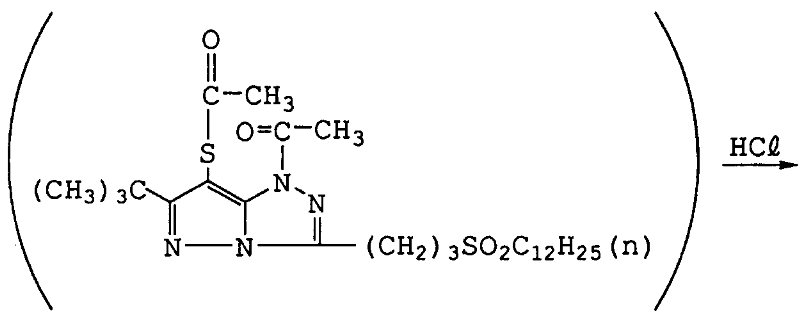


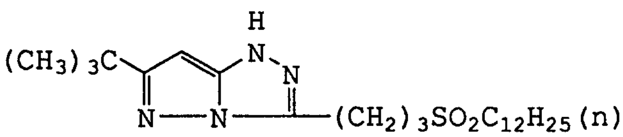

Exemplified compound [II]-7
(m.p. 116 to 117.5°C)

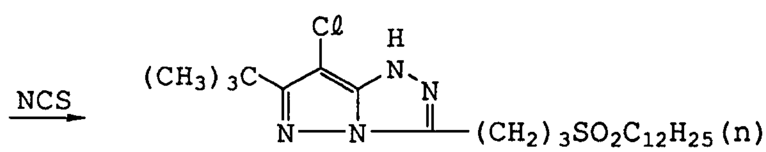


Exemplified compound [III]-21
(m.p. 65.5 to 66.5°C)

In a 2-liter three-necked flask were placed 200.0 g of the above compound A, the starting material, and 1,000 ml of acetic anhydride. The contents were refluxed with heating under a reduced pressure of 40 to 50 mm Hg, while removing about 800 ml of acetic acid in 2 hours. After confirming the termination of the reaction, the reaction mixture was cooled to about 70°C, 154 ml of water added thereto, then 176 ml of concentrated hydrochloric acid was added dropwise.

After the generation of heat terminated, the reaction mixture was refluxed by heating for 1 hour, cooled in the air, and precipitated granular sulfur was filtered. To the filtrate was added 2 liters of ethyl acetate, and the pH of the water layer was adjusted to about 5 with an aqueous solution of sodium carbonate, then it was washed with a saturated saline solution.

The organic layer was separated and dried with anhydrous magnesium sulfate, followed by removal of the ethyl acetate. Obtained were 182.8 g of light brown crystals of compound [II]-7. This compound gave a melting point of 116°C to 117.5°C after recrystallization from a 10:1 methanol:water mixed solvent.

After dissolving 100.0 g of the recrystallized compound [II] in 800 ml of ethyl acetate, N-chlorosuccinimide (NCS) was added thereto at room temperature in 20 minutes. The contents were stirred for 2 hours at room temperature, the subsequent removal of the ethyl acetate gave 100.7 g of crude crystals of compound [III]-21. The melting point of the compound after recrystallization was 65.5 to 66.5°C.

Example 1

The crude crystals of exemplified compound [II] − 7 prepared in synthesis example 1 were divided into portions of 10.0 g each: one subjected to no treatment after the synthesis (comparative sample 1), ones each recrystallized from a solvent independent of the invention (comparative samples 2 to 5), and ones each recrystallized from a recrystallizing solvent containing the aromatic hydrocarbon solvent of the invention (inventive samples 1 to 7). The purity and the taking − up yield (the weight ratio of a recrystallized sample to comparative sample 1) of each sample are shown in Table 1.

The sulfur contained in these couplers was determined in the following manner.

Determination of Sulfur

(1) Into a 10 − ml measuring flask was accurately weighed out 1.0 g of a coupler for quantitification. After adding 1 ml of toluene, the solution was accurately diluted to 10 ml with methanol.
(2) The solution prepared in (1) is subjected to quantitative analysis by the absolute calibration curve method using a high performance liquid chromatograph.

The high performance liquid chromatograph used was as follow:

| | |
|---|---|
| Pump : | 880 − PU (made by Nippin Bunko Co.) |
| Detector : | 870 − UV (made by Nippon Bunko Co.) |
| Column oven: | 860 − CO (made by Nippon Bunko Co.) |
| Integrator : | Love Chart 180z (made by System Instruments Co.) |
| Injector : | Leodyn 7125 (made by Leodyn) |

The detecting conditions were as follows:

| | |
|---|---|
| Column : | ODS − 2 (4.6 mm in diameter and 250 mm long, made by GL Science Co.) |
| Eluent : | acetonitrile/water = 80/20 |
| Flow rate : | 1.0 ml/min |
| Detecting wavelength: | 254 nm |
| Temperature : | 40 ° C |
| Amount injected : | 20 ml |

The purity of each of samples of exemplified compound [II] − 7 was determined by the simple area percentage method with the above liquid chromatograph used for the sulfur quantification. The detecting conditions and the sample solution used were as follows:

Detecting conditions

| | |
|---|---|
| Column : | ODS − 2 (4.6 mm in diameter and 250 mm long, made by GL Science Co.) |
| Eluent : | acetonitrile/water = 90/10 |
| Flow rate : | 1.0 ml/min |
| Detecting wavelength: | 254 nm |
| Temperature : | 40 ° C |
| Amount injected : | 20 ml |

Sample solution

Into a 10 − ml measuring flask was accurately weighed out 10 mg of a coupler, then an eluent was added to make the total volume exactly 10 ml.

Table 1

| Sample No. | Recrystallizing Solvent | Amount of Recrystallizing Solvent (ml) | Amount of Sulfur Detected (ppm) | Purity of Compound (%) | Taking-up Yield against Comp. Sample 1 (%) |
|---|---|---|---|---|---|
| Comp. sample 1 | no recrystallization | - | 2624 | 97.1 | 100 |
| Comp. sample 2 | methanol:water = 9:1 | 20 | 1800 | 97.3 | 91 |
| Comp. sample 3 | methanol | 15 | 1225 | 97.9 | 82 |
| Comp. sample 4 | acetonitrile | 30 | 516 | 98.2 | 81 |
| Comp. sample 5 | ethyl acetate | 40 | 123 | 98.3 | 76 |
| Inventive sample 1 | ethyl acetate:toluene = 8:1 | 45 | 19.7 | 98.7 | 77 |
| Inventive sample 2 | ethyl acetate:toluene = 2:1 | 30 | 11.3 | 99.1 | 84 |
| Inventive sample 3 | ethyl acetate:toluene = 1:9 | 30 | 2.1 | 99.2 | 92 |
| Inventive sample 4 | toluene | 30 | 0 | 99.8 | 97 |
| Inventive sample 5 | toluene | 20 | 0.3 | 99.5 | 97 |
| Inventive sample 6 | acetonitrile:benzene = 9:1 | 20 | 25.1 | 99.3 | 79 |
| Inventive sample 7 | ethyl acetate:xylene = 3:1 | 30 | 16.4 | 99.3 | 81 |

Example 2

The crude crystals of exemplified compound [III] − 21 obtained in synthesis example 1 were divided into portions of 10.0 g each: one subjected to no treatment after the synthesis (comparative sample 6), ones

each recrystallized from a solvent independent of the invention (comparative samples 7 and 8), and ones each recrystallized from a recrystallizing solvent containing the aromatic hydrocarbon solvent of the invention (inventive samples 8 to 10). The purity and the taking-up yield of compound [III]-21 (the weight ratio of a recrystallized sample to comparative sample 8) are shown in table 2 note that the purity of each coupler sample and sulfer content were determined in the same manner as in example 1.

As shown in Tables 1 and 2, the invention can give the objective compound of high purity, with an extremely low sulfur content in pyrazolo[3,2-c]-1,2,4-triazole compounds used as a coupler, without lowering the yield.

Table 2

| Sample No. | Recrystallizing Solvent | Amount of Recrystallizing Solvent (ml) | Amount od Sulfur Detected (ppm) | Purity of Compound (%) | Taking-up Yield against Comp. Sample 1 (%) |
|---|---|---|---|---|---|
| Comp. sample 6 | no recrystallization | - | 250 | 97.4 | 100 |
| Comp. sample 7 | acetonitrile | 50 | 49 | 97.8 | 71 |
| Comp. sample 8 | methanol | 30 | 113 | 98.2 | 95 |
| Inventive sample 8 | methanol:toluene = 19:1 | 30 | 5.6 | 99.7 | 93 |
| Inventive sample 9 | methanol:toluene = 9:1 | 30 | 2.1 | 99.7 | 89 |
| Inventive sample 10 | acetonitrile:toluene = 19:1 | 40 | 1.0 | 99.1 | 70 |

As described above, pyrazolo[3,2-c]-1,2,4-triazole compounds, which are useful as photographic couplers or intermediates thereof and as intermediates in various organic syntheses, can be produced in a high purity by the process according to the invention.

## Claims

1. In a process of manufacturing a pyrazolo[3,2-c]-1,2,4-triazole compound represented by Formula [II] or [III] from a 1,2,4-triazolo[3,4-b]-1,3,4-thiadiazine compound represented by Formula [I] as an intermediate, wherein a crude product of said pyrazolo[3,2-c]-1,2,4-triazole compound is contaminated by sulfur as an impurity, which is a by-product formed in the process of manufacture,
   the improvement comprising:
   removing said sulfur by recrystallizing the product from a solution containing an aromatic hydrocarbon solvent,
   Formula [I]

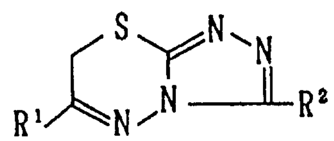

   Formula [II]

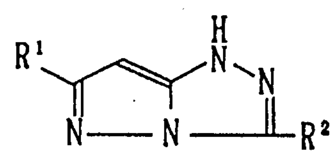

   Formula [III]

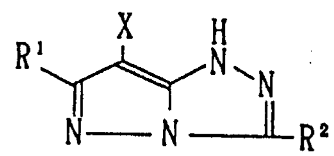

   wherein $R^1$ and $R^2$ each represent a hydrogen atom or a substituent, and X represents a substituent capable of being split off upon reaction with an oxidation product of a color developing agent.

2. The process of claim 1, wherein said aromatic hydrocarbon solvent is benzene, toluene, xylene, chlorobenzene, trichlorobenzene or nitrobenzene.